# EUROPEAN PATENT APPLICATION

(11) **EP 2 228 014 A1**
(43) Date of publication of application: **15.09.2010**
(21) Application number: 09155164.8
(22) Date of filing: 13.03.2009
(51) Int. Cl.: A61B 17/02, A61B 17/34

(54) **Rib retractor**

(71) Applicant: Cardio Life Research S.A., 1930 Zaventem (BE)
(72) Inventor: Joie, Michel, 5030, ERNAGE (BE)
(74) Representative: Vandeberg, Marie-Paule L.G.

(57) **Abstract**

A rib retractor (1) comprises a pair of L-shaped members (2, 4) able to be pivoted between an inserting and a working shape. Each member (2, 4) comprises a detachable handle (6) at a proximal side and a stretching jaw (18) at a distal side. The members (2, 4) are linked at their corners by pivoting joining means (10). Each stretching jaw (18) comprises a distal blade part (16) and a curved section (18), the latter acting as a cam to stretch apart the ribs (14) when the retractor (1) is brought from inserting to working shape and which define the operating field.

## Description

### Field of the invention

The invention relates to a device for moving apart the ribs of the thoracic cage in order to improve accessibility to internal organs of a patient, for example for thoracic and particularly heart surgery.

### Description of prior art

A known procedure to move ribs apart comprises the following steps: making an incision between two ribs, inserting between the ribs a ring the diameter of which is larger than the intercostal space, then pulling on strips fastened to said ring, so as to pull the ribs apart. This method implies fastening the strips, which can be tedious.

A device for providing the same function are known from US- 4 627 421. This document describes a retractor with pivoting arms. One arm is mounted on a teethed cross-bar and the other is mounted on a cursor that moves along the cross-bar.

Other similar devices are described in WO 99/15081, US-2001/0041828, DE-3834358.

All these devices are particularly aimed at classic thoracic surgery, meaning that the ribs are submitted to a strong pulling-apart force, and accordingly, less adapted to mini-invasive surgery. Mini-invasive surgery reduces to a minimum the wounds and bruises the body of the patient has to undergo and hence ensures a quicker recovery.

### Summary of the invention

It is an object of the invention to provide a rib retractor that would provide a good access to organs placed in the ribcage.

Another object of the invention is to reduce as much as possible the strains exerted on the ribs.

Still another object of the invention is to provide a rib retractor of reduced cumbersomeness and easy to use.

The subject of the invention is a rib retractor which comprises two members extending from a proximal to a distal side. Each member comprises a stretching jaw and a handle. The stretching jaw is placed at the distal side and comprises:
- a substantially flat blade section having a free end and an opposite end, which is permanently connected to
- a curved section extending substantially normal to the blade section, the convexity of said curved section directed towards the free end of the blade section.
   The handle is detachably connected to the proximal side of the curved section of the stretching jaw and extends normal to the blade thereof, prolonging the curved section.
   The two members are pivotally connectable between an inserting and a working position. In the inserting position, the two blades of the stretching jaws are substantially in contact with and facing each other, allowing insertion thereof between two ribs. In the working position, the curved sections of the jaws face each other, defining an operating field. The curved sections of the jaws provoke, upon pivoting of the members from the inserting to the working position, a cam effect, thus pushing the ribs apart.

According to an advantageous embodiment, the two members are pivotally connected by detachable ball-and socket joint means placed on either sides of the concave side of the curved sections of the jaws.

According to an advantageous embodiment, the two members are pivotally connected by detachable mutually engaging gearings placed on either sides of the concave side of the curved sections of the jaws.

Each curved central part is preferably provided at both lateral sides with a sheath, the corresponding sheaths of each central part lying parallel to each other when the rib retractor is in working position.

According to an advantageous embodiment, U-form securing bridges able to be inserted in the corresponding sheaths of each central part so as to lock them in working position are provided. These U-form securing bridges preferably comprise support means for securing other medical devices, tubes, sutures and so on.

Advantageously, the rib retractor comprises a screw extension device comprising a threaded rod and two members able to be moved relative to each other along said threaded rod, each of these members being able to be inserted into the corresponding sheaths on at least one side of each central part so as to pull them apart. Bridges of various lengths are then provided.

According to another embodiment, the rib retractor comprises a ratchet extension device comprising a toothed rod and two members able to be moved relative to each other along said toothed rod, each of these members being able to be inserted into the corresponding sheaths on at least one side of each central part so as to pull them apart.

The curved sections preferably have an U-shaped cross-section, the open side of the U being directed opposite to the free end of the substantially flat blade section.

The handles and the stretching jaw are advantageously linked by mortice and tenon joint means.

The members are made preferably out of polymer materials, and are advantageously disposable.

Each member preferably comprises a pad, preferably made out of a soft material, as e.g. silicone, extending along the convex side of the curved section and the blade part.

This pad advantageously comprises a haemostatic material.

### Short description of the drawings

These and further aspects of the invention will be explained in greater detail by way of example and with reference to the accompanying drawings in which:
- Fig.1: is a view in perspective of a rib retractor according to the invention in working position;
- Fig.2a: is a sketch cross-section view along plane II-II of the rib retractor of Fig.1 in working position or shape;
- Fig.2b: is a sketch cross-section view of the rib retractor of Fig.2a in inserting position or shape;
- Fig.3 to 7: form a series of views in perspective of various stages in the use of a rib retractor of the invention;

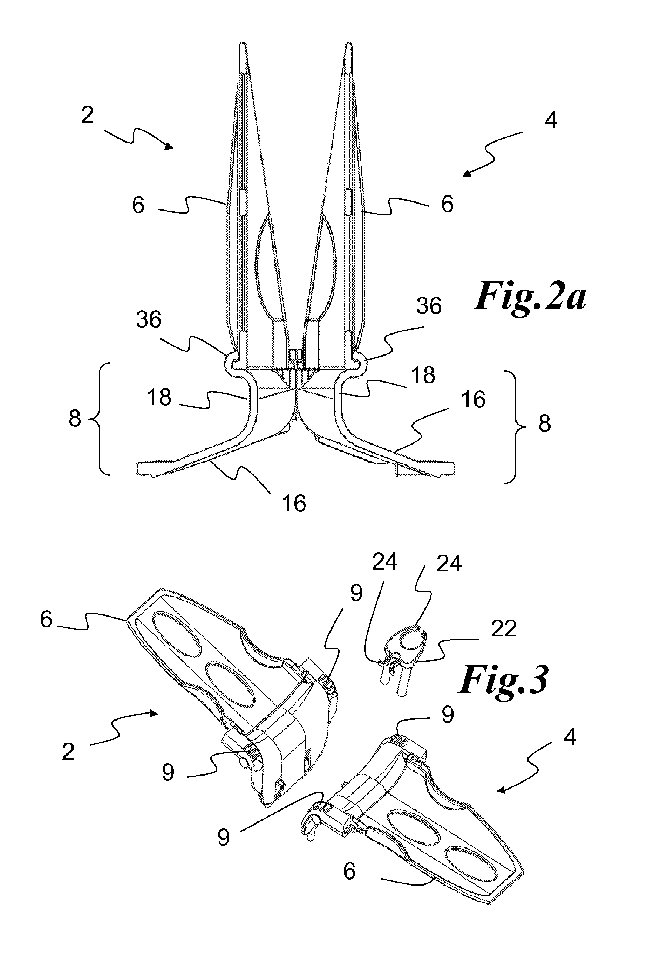

- Fig. 3: is a view in perspective of the not yet assembled retractor;

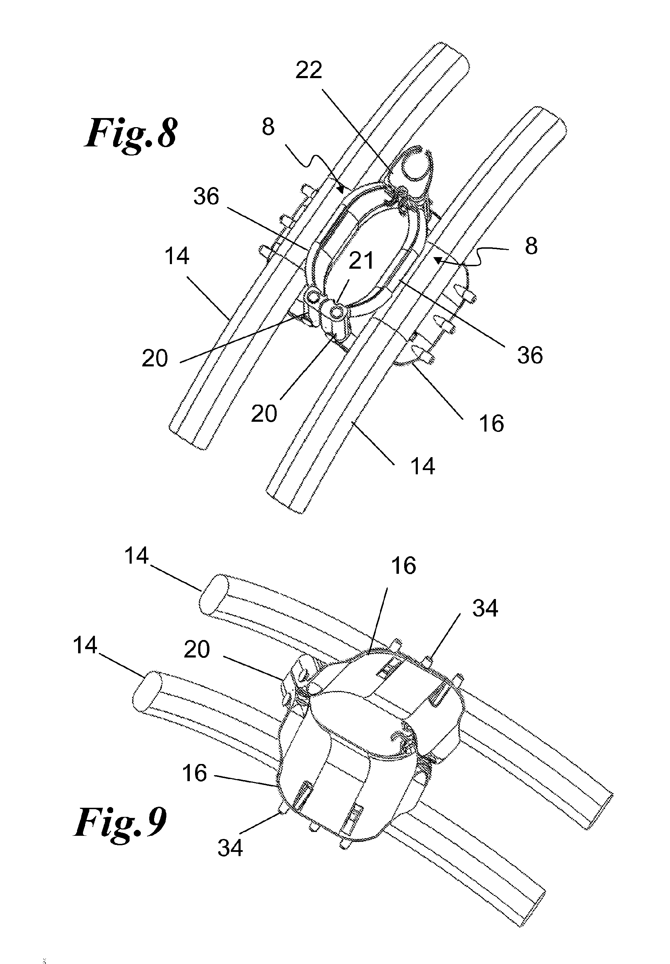

- Fig. 8 and 9: are sketch views in perspective of the jaws of the rib retractor in working position between two ribs;

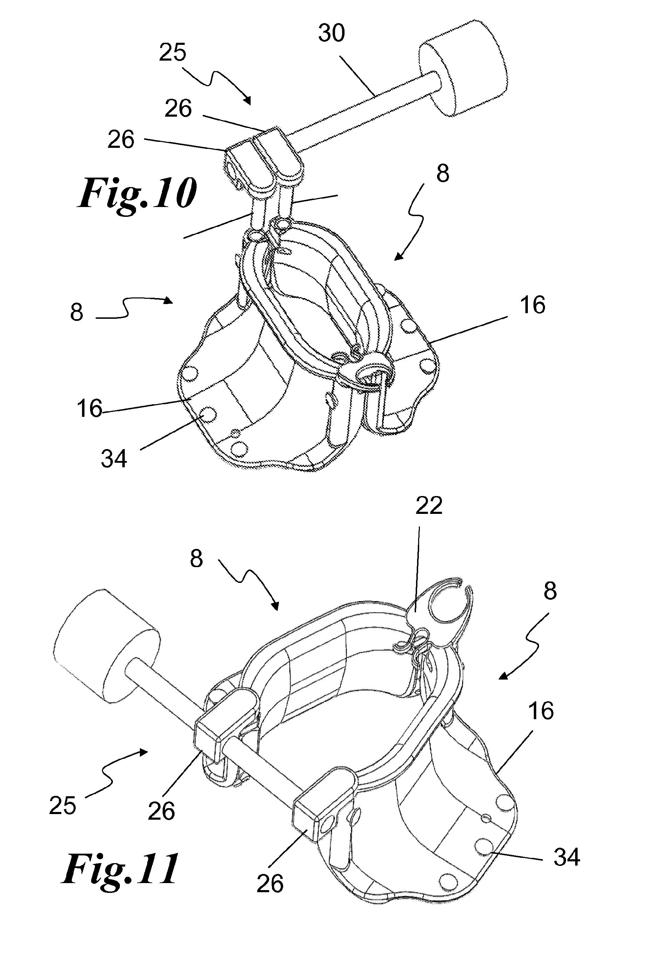

- Fig. 10 and 11: are sketch views in perspective of the jaws of a retractor according to the invention further comprising a stretching device;

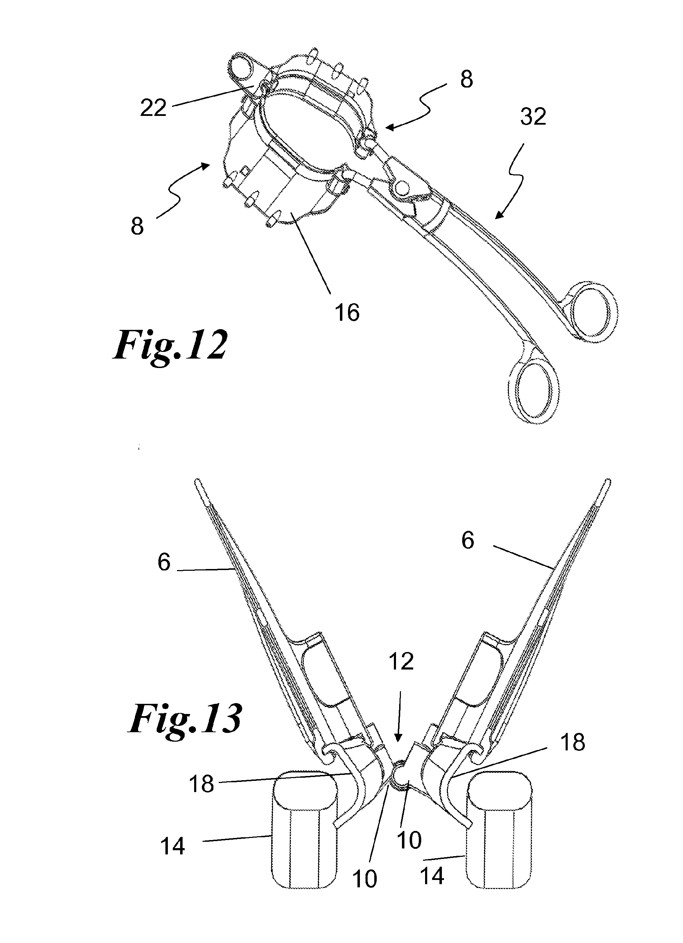

- Fig.12: is a view in perspective of the jaws of a retractor according to the invention further comprising another embodiment of stretching device;

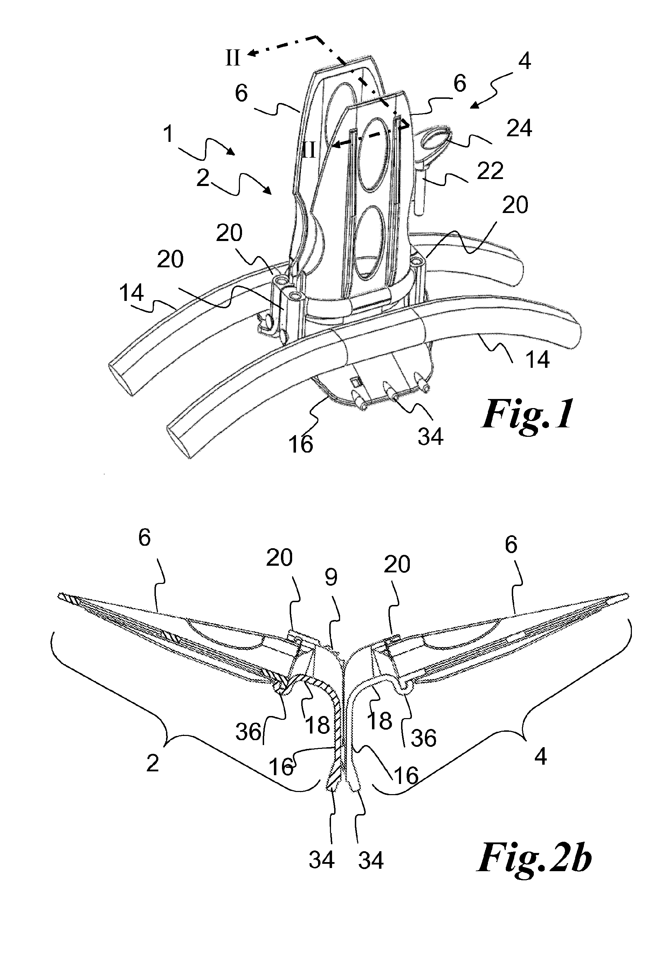

- Fig.13: is a cross-section view along plane II-II of another embodiment the rib retractor of Fig.1 in an intermediary position;

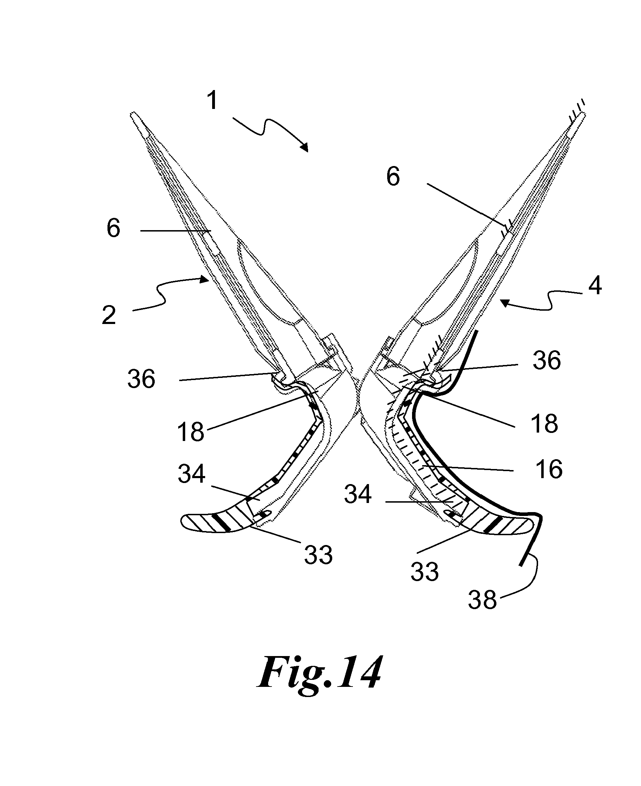

- Fig.14: is a cross-section view along plane II-II of the rib retractor of Fig.1 in an intermediary position further comprising a soft pad.

The figures are not drawn to scale. Generally, identical components are denoted by the same reference numerals in the figures.

### Detailed description of preferred embodiments

As can be seen from Fig. 1, 2a and 2b, the rib retractor of the invention 1 is formed of a pair of members 2, 4, each member having substantially, in transversal cross-section, a L-shape, the poles of the L extending parallel to each other when the retractor 1 is in working position (Fig 1 and 2a).

The upper or proximal parts of the poles of the L constitute the handles 6 of the rib retractor 1. The lower or distal section of the poles, together with the base of the L, constitute the head section or the "jaws" 8 of the retractor 1, as this retractor can be considered as a kind of pliers. Mutually engaging gearings 9 extending along both sides of the jaws 8 form pivoting connections. By acting on the handles 6, the operator can force the members 2, 4 to pivot around the pivoting axes determined by the two pivoting connections, bringing the jaws 8 of the head section from an insertion to a working shape or position and reversely.

One notes that when the retractor is in its insertion position (Fig.2b), the bases of the L-shaped members, which have the form of substantially flat blades 16 lean close to each other, so that they can be easily inserted between the ribs 14 of a ribcage.

As apparent from Fig. 1 to 2b, the pole part of the L forming each member 2, 4 is curved, the cave sides of the curves being directed towards each other.

The use of the retractor 1 will become more obvious when looking at the series of Fig. 3 to 7.

In the embodiment displayed at Fig.3, the mutually engaging gearings 32 are detachable from each other. Accordingly, the two members 2, 4 may be assembled at the very moment when the retractor 1 is put in service, meaning that it can be stocked unassembled in a very small sterile packaging.

The two members 2, 4 having been put together, the retractor 1 is inserted, in its inserting shape, between two ribs 14, through a relatively small hole cut between the required ribs of the patient. As represented in Fig.2b, the blades 16 extending at the distal side of the retractor are at this stage close to each other and can thus be inserted easily between the edges of the incision.

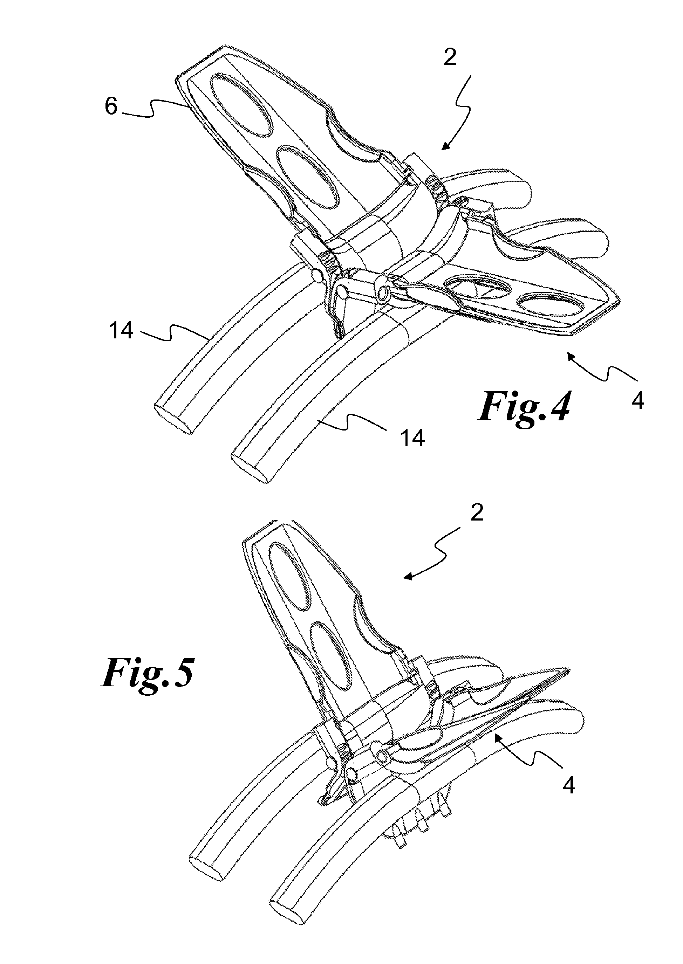

As shown at Fig.5, the surgeon then applies a closing force on the handles 6. This causes first the blades 16 to move around the ribs 14, ensuring a good fastening of the retractor, but also the curved section 18 of the jaws to exert a cam effect on the ribs 14, forcing them apart.

The rib retractor having reached its working shape, the side parts of each curved section 18 of one jaw press against the corresponding side part of the other curved section 18. They thus form together a steady ring which will delimitate the operating field.

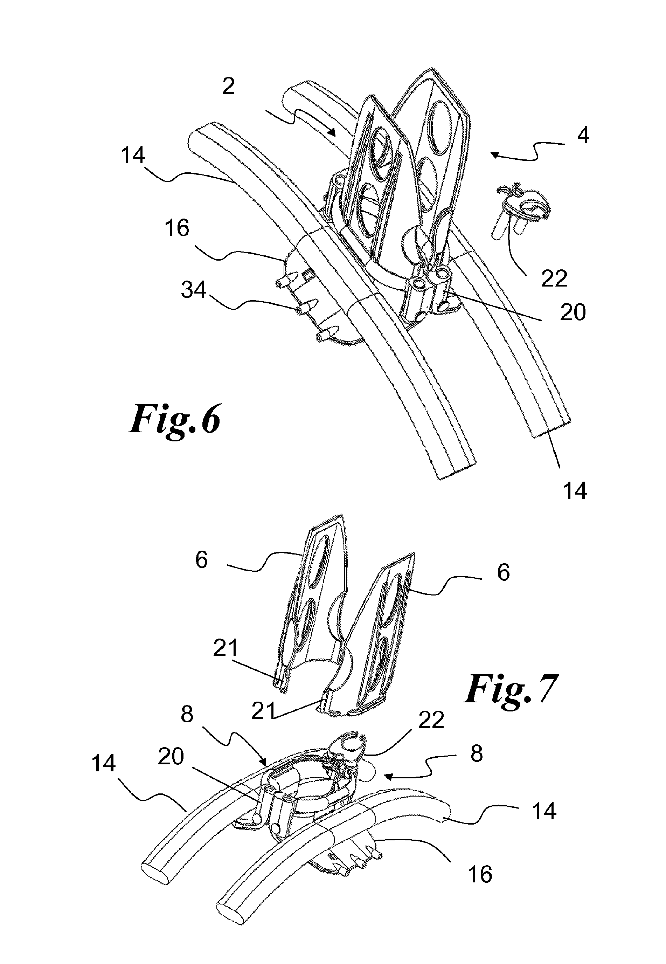

Each curved section 18 is provided at both lateral sides with sheaths 20. As represented at Fig.6 the corresponding sheaths 20 of each curved section 18 lie at this moment (i.e. when the rib retractor is in working shape) parallel to each other.

To reinforce the stability of this ring, U-form securing bridges 22 are then inserted into the adjoining sheaths 20.
The curved sections 18 forming now a mechanically continuous ring, the handles 6, fastened to the jaws by known mortice and tenon joint means 21, can be removed (see Fig.7).

It will be noted that the slight curvature of the handles 6 not only allow them to match the curvature of the curved section 18 of the jaws, but also contributes to ensures their mechanical strength. An advantage of the present retractor 1 over prior art devices is that the surgeon can estimate, from the effort he has to exert on the handles 6, the stress the ribs 14 undergo when pulled apart.

Fig.8 and 9 are artist's views of the fastened jaws seen from various points of view. The securing bridges 22 comprise support means 24 for securing possible other medical devices as catheters, sucking tubes, suturing wires (the support means acting then as suture organizers), an apparatus as described in WO 2006/000514 and so on.

Fig.10 and 11 display an advantageous additional feature 25 that can be used with the retractor 1 of the invention: if the surgeon is of opinion that the ribs 14 should be moved further apart, he can remove one of the securing bridges 22 and replace it by bolts 26 provided with an adequate inserting pin 28, allowing them to be fastened to the sheaths 20 on one side of the jaws 18. These bolts are movable relative to each other along a threaded shaft 30, as represented, or along a (not represented) ratchet. Rotating the shaft 30 provokes a pulling apart on one side of the jaws 18, the other side of the jaws rotating around the pins of the remaining securing bridge 22. If needed, both securing bridges 22 can be replaced by such pulling-apart features 25. Those features, contrary to other parts of the retractor, are advantageously made out of metal, while other parts of the retractor are preferably made out of disposable materials as polymers, for sake of sterility. The rib retractor according to the invention can also be provided with a series of bridges 22 of increasing lengths. If, for some reason, the surgeon wants to widen the operating field, he can remove one of the initial bridges 22 and exert a pulling (using a lever or specially designed forceps 32), as represented at Fig. 12, between the jaws 18, which can then be maintained apart by a longer (not represented) bridge 22.

Fig. 13 displays another embodiment of the retractor wherein the mutually engaging gearings 9 extending along both sides of the curved sections 18 are replaced by ball-and socket joint means. Two parts 10 protruding between the jaws 8 on either sides of each curved section 18 form a pivoting connection 12.

As can be seen throughout the figures, the free ends of the blade parts are provided with fastening means 34, allowing the fastening thereon of pads of soft materials 33(as shown at Fig.14), which spread the stress exerted on the ribs 14 and the surrounding tissues, minimizing i.a. the risk of bruising the ribs 14. A layer of haemostatic material 38 preventing blood to pour out into the operating field during the operation can also be provided.

As particularly apparent from figure 14, the curved part 18 of the jaws 8 has a U-shaped cross-section. Accordingly, when the rib retractor is in working position, the upper (proximal) part of the curved section 18, forming a lip 36, rests on the outer side of the ribs 14, ensuring a better stability of the ring.

It will be appreciated by persons skilled in the art that the present invention is not limited by what has been particularly shown and described hereinabove. The invention resides in each and every novel characteristic feature and each and every combination of characteristic features. Reference numerals in the claims do not limit their protective scope. Use of the verb "to comprise" and its conjugations does not exclude the presence of elements other than those stated. Use of the article "a" or "an" preceding an element does not exclude the presence of a plurality of such elements.

The present invention has been described in terms of specific embodiments, which are illustrative of the invention and not to be construed as limiting.

## Claims

1. A rib retractor (1) comprising two members (2, 4), extending from a proximal to a distal side, each member comprising:
(a) a stretching jaw (8), placed at the distal side, comprising:
- a substantially flat blade section (16), having a free end and an opposite end, which is permanently connected to
- a curved section (18) extending substantially normal to the blade section (16), the convexity of said curved section directed towards the free end of the blade section; and
(b) a handle (6) detachably connected to the proximal side of the curved section (18) of the stretching jaw (8), extending normal to the blade (16) thereof and prolonging the curved section (18);
the two members (2, 4) being pivotally connectable between an inserting and a working position, wherein
- in the inserting position, the two blades (16) of the stretching jaws (8) are substantially in contact with and facing each other, allowing insertion thereof between two ribs (14), and
- in the working position, the curved sections of the jaws face each other, defining an operating field,
the curved sections (18) of the jaws (8) provoking, upon pivoting of the members (2, 4) from the inserting to the working position, a cam effect, thus pushing the ribs (14) apart.

2. A rib retractor according to claim 1, **characterized in that** the two members are pivotally connected by detachable ball-and socket joint means placed on either sides of the concave side of the curved sections (18) of the jaws (8).

3. A rib retractor according to claim 1,
**characterized in that** the two members are pivotally connected by detachable mutually engaging gearings placed on either sides of the concave side of the curved sections (18) of the jaws (8).

4. A rib retractor according to any one of preceding claims **characterized in that** each curved central part (18) is provided at both lateral sides with a sheath (20), the corresponding sheaths (20) of each curved section (18) lying parallel to each other when the rib retractor (1) is in working position

5. A rib retractor according to claim 4
**characterized in that** it further comprises U-form securing bridges (22) able to be inserted in the corresponding sheaths (20) of each curved section (18) so as to lock them in working position.

6. A rib retractor according to claim 5
**characterized in that** the U-form securing bridges (22) further comprise support means (24) for securing other medical devices.

7. A rib retractor according to any one of claims 4 to 6 **characterized in that** it further comprises a screw or ratchet extension device (25) comprising a rod and two members (26) able to be moved relative to each other along said rod, each of these members being able to be inserted into the corresponding sheaths (20) on at least one side of each curved section (18) so as to pull them apart.

8. A rib retractor according to any one of claims 5 to 7 **characterized in that** bridges (22) of various lengths are provided.

9. A rib retractor according to any one of the preceding claims **characterized in that** the curved sections (18) have a U-shaped cross-section, the open side of the U, forming a lip (36) being directed opposite to the free end of the substantially flat blade section (16).

10. A rib retractor according to any one of the preceding claims **characterized in that** the handles (6) and the stretching jaw (18) are linked by mortice and tenon joint means (21).

11. A rib retractor according to any one of the preceding claims **characterized in that** the members (2, 4) are made out of polymer material.

12. A rib retractor according to any one of the preceding claims **characterized in that** the members (2, 4) are disposable.

13. A rib retractor according to any one of the preceding claims **characterized in that** each member (2, 4) further comprises a pad extending along the convex side of the curved section and the blade part.

14. A rib retractor according to claim 13 **characterized in that** the pad is made out of a soft material.

15. A rib retractor according to claim 13
**characterized in that** the pad comprises a haemostatic material.
